# EUROPEAN PATENT SPECIFICATION

(11) **EP 3 781 261 B1**
(45) Date of publication and mention of the grant of the patent: **26.11.2025**
(21) Application number: 19836965.4
(22) Date of filing: 08.03.2019
(51) Int. Cl.: A61K 31/4985, A61K 9/48, A61P 3/10

(54) **ORAL PHARMACEUTICAL COMPOSITIONS COMPRISING DPP-4 INHIBITOR**
ORALE PHARMAZEUTISCHE ZUSAMMENSETZUNGEN MIT DPP-4-INHIBITOR
COMPOSITIONS PHARMACEUTIQUES ORALES COMPRENANT UN INHIBITEUR DE DPP-4

(30) Priority: 17.04.2018 TR 201805452
(43) Date of publication of application: 24.02.2021
(73) Proprietor: Sanovel Ilac Sanayi ve Ticaret A.S., Sisli - Istanbul (TR)
(72) Inventor: TURKYILMAZ, Ali, 34460 Istanbul (TR); ATAMAN, Seval, 34460 Istanbul (TR); ERKAY, Cagil, 34460 Istanbul (TR)
(74) Representative: Genç Ilhan, Oznur
(86) International application number: PCT/TR2019/050144
(87) International publication number: WO 2020/018034

(56) References cited:
- WO-A2-2010/000469
- WO-A2-2019/203771
- US-A1- 2016 367 552
- SWAMINATHAN, V. ET AL.: "An examination of the moisture sorption characteristics of commercial magnesium stearate", AAPS PHARMSCITECH, vol. 2, no. 4, 2001, pages 73, XP036412799, Retrieved from the Internet <URL:https://doi.org/10.1007/BF02830568> DOI: 10.1208/pt020428

## Description

### Field of the Invention

The present invention relates to solid oral pharmaceutical compositions comprising at least one DPP-4 inhibitor, particularly sitagliptin, or a pharmaceutically acceptable salt thereof, providing ease of manufacture and high stability.

### Background of the Invention

Type 2 Diabetes Mellitus (T2DM) is an epidemic disease that is widespread worldwide and has important consequences for patients and even for the society. In healthy individuals, immediately after oral glucose administration, depot insulin (first phase insulin release), previously produced by pancreatic beta cells, is secreted. Subsequently, insulin produced in β cells (2nd phase insulin release) is used. Increased insulin response to hyperglycemia after oral glucose uptake is called incretin effect. The incretin hormones, glucagon-like peptide 1 (GLP-1) and gastric inhibitor polypeptide (GIP), play an important role in glucose homeostasis in healthy individuals by stimulating postprandial pancreatic insulin secretion and inhibiting glucagon release.

However, in patients with T2DM, the insulin response that is supposed to increase after carbohydrate intake is reduced or delayed, glucagon release is increased, resulting in postprandial hyperglycaemia. In other words, GLP-1-activated pancreatic insulin release is insufficient, insulinotropic effects of the incretin hormones in the peptide structure are diminished in patients with T2DM. Because both incretins are cleaved by dipeptidyl peptidase-4 (DPP-4) enzyme in minutes. In diabetic patients, DPP-4 cleaves and inactivates GLP-1, the synthesis of which is increased due to elevated postprandial plasma glucose levels, resulting in disrupted balance in blood-glucose levels. In order to stabilize glucose levels, DPP-4 inhibitors inhibit the DPP-4 enzyme that cleaves and inactivate the incretin hormones released by the small intestine, causing elevated GLP-1 and GIP levels and lower glucose and ultimately glycosylated hemoglobin (HbA1c) levels in patients

In the art, many DPP-4 inhibitors have been developed, mainly sitagliptin, vildagliptin, saxagliptin, alogliptin, denagliptin, ASP8497.

Different dosage forms comprising DPP-4 inhibitors are also disclosed in patent documents in the prior art. For example, in a document addressing the problem of mechanical strength of immediate-release tablet forms comprising DPP-4 inhibitors, it is aimed to develop a formulation that would not reduce release rate or tablet strength by increasing porosity excessively. In addition, it is stated that 20-60% of diabetic patients also had hypertension and that should be taken into account when designing tablet formulations. Accordingly, tablet formulations with sodium content of less than 10 mg and potassium content of less than 15 mg have been proposed.

The chemical name of sitagliptin is (R) -4-oxo-4- [3- (trifluoromethyl) -5,6-dihydro [1,2,4] triazolo [4,3-a] pyrazin-7 (8H)-yl]-1- (2,4,5-trifluorophenyl) butan-2-amine), and the chemical structure of sitagliptin is shown in Formula 1.

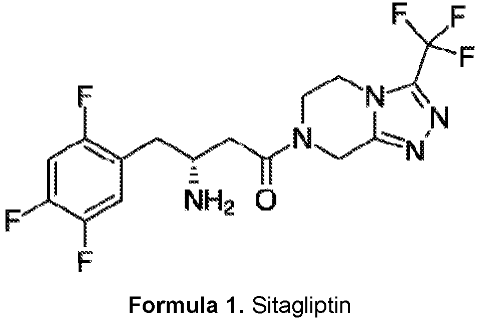

In current T2DM treatments, sitagliptin is used alone or in combination with other oral antidiabetics. In order to provide high solubility and stability in compositions available in the art, sitagliptin is usually in the form of salts. A formulation in which sitagliptin phosphate monohydrate salt is used is registered under the trade name of JANUVIA^{®}.

The documents in prior art also disclose exemplary formulations where the hydrochloride salt of sitagliptin is used. In one of these examples, a tablet formulation comprising sitagliptin hydrochloride monohydrate salt has been developed. The goal in the development of said formulation is to increase the stability of the final dosage form by lowering the impurity ratio to below 5%. However, it is known that the moisture retention capacity of sitagliptin HCl salt is 3-10 times higher than other salts of sitagliptin. It is clear that this will adversely affect the tablet stability.

One of the two major problems with the solid oral dosage formulations of DPP-4 inhibitors is the difficulty of maintaining the stability due to the sensitivity of the product to moisture, and the other is the reduced bioavailability due to decreased solubility caused by excipients used to reduce moisture retention. In the art, studies have also been carried out to improve the bioavailability. For example, a formulation using the amorphous form of sitagliptin comprises polyvinylpyrrolidone as crystallization inhibitor to improve bioavailability, which also acts as a binder. However, highly hygroscopic feature of polyvinylpyrrolidone is disclosed in available sources (Handbook of Pharmaceutical Excipients, R. C. Rowe, P. J. Sheskey and M.E. Quinn, sixth edition, page 582).

In formulations comprising a DPP-4 inhibitor, use of the binder is presented as a requirement for the stability of the final product. Furthermore, magnesium stearate having both glidant and lubricant properties is widely used to achieve flowability. However, highly hygroscopic feature of magnesium stearate, just like that of polyvinylpyrrolidone, is an obstacle in way of forming stable formulations.

Based on these information, there is a need in the art for solid oral pharmaceutical compositions with high stability and bioavailability by selecting appropriate forms of DPP-4 inhibitors and combining them with suitable excipients without using any binders.

### Objectives and Brief Description of the Invention

The main object of the present invention is to obtain solid oral pharmaceutical compositions comprising at least one DPP-4 inhibitor, particularly sitagliptin, which removes all the above-mentioned problems and brings additional advantages to the prior art.

A further object of the present invention is to develop solid oral pharmaceutical compositions comprising sitagliptin and having low moisture retention.

A further object of the present invention is to develop solid oral pharmaceutical compositions comprising sitagliptin and having improved stability and prolonged shelf life.

Another object of the present invention is to develop solid oral pharmaceutical compositions comprising sitagliptin and having high flowability.

A further object of the present invention is to develop solid oral pharmaceutical compositions comprising sitagliptin and having a high dissolution rate, high solubility and thus high bioavailability.

In the art, the patent application numbered US 2016/367552 A1 discloses a film coated tablet comprising sitagliptin, microcrystalline cellulose, dibasic calcium phosphate anhydrous, croscarmellose sodium, citric acid, magnesium stearate, sodium stearyl fumarate, colloidal silicon dioxide and opadry II. Although said formulation does not contain binder, it contains a considerable amount of magnesium stearate. On the other hand, sitagliptin in its free base form is used and it is not associated with the absence of a binder. The patent application WO 2010/000469 A2 teaches crystalline monobasic, dibasic and tribasic acid addition salts of sitagliptin including crystalline forms of sitagliptin maleate.

Another object of the present invention is to develop solid oral pharmaceutical compositions comprising sitagliptin for use in the treatment of type 2 diabetic patients, that also have safe components for use in hypertensive and cardiac patients.

Another object of the present invention is to develop solid oral pharmaceutical compositions of sitagliptin with the above-mentioned properties.

Another object of the present invention is to develop capsule dosage forms of sitagliptin with high moisture resistance.

### Detailed Description of the Invention

The invention is defined in the claims.

For the purposes outlined above, detailed features of the present invention are presented herein.

The present invention relates to a solid oral pharmaceutical composition comprising at least one DPP-4 inhibitor or a pharmaceutically acceptable salt thereof, wherein the composition is free of polyvinylpyrrolidone and wherein said DPP-4 inhibitor or a pharmaceutically acceptable salt thereof is sitagliptin maleate.

According to a preferred embodiment of the invention, the composition subjected to the invention is also free of magnesium stearate.

Magnesium stearate serves both as glidant and lubricant in solid oral pharmaceutical formulations. Although providing high flowability in formulations, magnesium stearate has high moisture retention and increases destabilization rate by increasing degradation, these characteristics make it less preferable especially for moisture-sensitive formulations.

According to one embodiment of the invention, DPP-4 inhibitor or a pharmaceutically acceptable salt thereof is in the form of sitagliptin maleate.

According to one embodiment of the invention, sitagliptin maleate is in amorphous form or in polymorph form, preferably in polymorph form. According to the most preferred embodiment of the invention, the composition comprises at least one polymorph forms of sitagliptin maleate comprising Form I, Form II, Form 11, Form I2, Form 13, Form I4, Form I5 or mixtures thereof.

According to a most preferred embodiment, the composition comprises sitagliptin maleate in the polymorph Form II.

According to this preferred embodiment, the amount of sitagliptin maleate in the total composition is 5-60%, preferably 10-50%, most preferably 15-30% by weight.

Yet according to this embodiment, the amount of sitagliptin in the composition is between 1 mg and 300 mg, preferably between 10 mg and 240 mg and more preferably between 20 mg and 120 mg. Most preferably, the composition of the invention comprises 25 mg or 50 mg or 100 mg sitagliptin.

It has been observed that the use of sitagliptin maleate salt as the active ingredient increases both stability and solubility. Sitagliptin phosphate monohydrate has very low solubility and sitagliptin hydrochloride has very high moisture retention, making them non-desirable salts for the present invention.

The formulation of the invention was designed without having to compromise on stability to achieve high solubility and bioavailability, which was linked to the surprisingly coordinated effect of using sitagliptin maleate as a source of sitagliptin and the absence of magnesium stearate and polyvinylpyrrolidone in the formulation. Formulations prepared with sitagliptin phosphate monohydrate and sitagliptin hydrochloride did not have the same effect. The said surprising effect is further enhanced by using the polymorph form of sitagliptin maleate.

Another point to be noted when creating the formulation of the invention is that the final product obtained has safe components for use in hypertensive and cardiac patients. Because it is known that the vast majority of type 2 diabetic patients also have these diseases. It is known that excessive use of potassium causes heartbeat irregularities, excessive use of sodium triggers hypertension by increasing intravascular volume.

In the preferred embodiment of the invention, the composition does not comprise potassium. The amount of sodium is kept below 15%, more preferably below 10% by weight of the composition. In this regard it is possible to develop solid oral pharmaceutical compositions of sitagliptin having safe components for hypertensive and cardiac patients.

According to one embodiment, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film coated tablet, gastric disintegrating tablet, pill, capsule, oral granule, powder, coated bead system, microsphere, tablet in tablet, inlay tablet, dragee, sachet or orally administrable film.

The composition is preferably in the form of capsule.

In one embodiment of the invention, the composition further comprises at least one excipient selected from fillers, disintegrants, lubricants, glidants or mixtures thereof.

According to one embodiment of the invention, the composition comprises at least one filler selected from the group comprising microcrystalline cellulose, lactose, mannitol, spray dried mannitol, starch, dextrose, sucrose, fructose, maltose, sorbitol, xylitol, inositol, kaolin, inorganic salts, calcium salts, polysaccharides, dibasic calcium phosphate anhydrate, sodium chloride, dextrates, lactitol, maltodextrin , sucrose-maltodextrin mixture, trehalose, sodium carbonate, sodium bicarbonate, calcium carbonate polyols, dextrose, maltitol, or mixtures thereof.

In a preferred embodiment of the invention, the composition comprises two fillers which are microcrystalline cellulose and dibasic calcium phosphate anhydrate.

The amount of microcrystalline cellulose is in the range of 5-60%, preferably 10-50%, most preferably 20-40% by weight of the total composition.

The amount of dibasic calcium phosphate anhydrate is 10-70%, preferably 20-60%, most preferably 30-45% by weight of the total composition.

According to one embodiment of the invention, the composition comprises at least one disintegrant selected from the group comprising of croscarmellose sodium, sodium carbonate, hydroxypropyl cellulose (HPC), copovidone, polycarbophil, low substituted poloxamer, sodium starch glycollate, starch, pregelatinized starch, alginic acid and alginates, ion exchange resins, magnesium aluminum silicate, sodium dodecyl sulfate, sodium carboxy methyl cellulose, carboxy methyl cellulose calcium, sodium docusate, guar gum, sodium alginate, sodium glycine carbonate, sodium lauryl sulfate, or mixtures thereof.

Considering the moisture retention properties of the disintegrants; the amount of disintegrant is kept below 10%, more preferably below 7% by weight of the composition, in order to ensure continuity of stability. In a preferred embodiment of the invention, the composition comprises a single disintegrant which is croscarmellose sodium.

According to one embodiment of the invention, the composition comprises at least one lubricant and at least one glidant selected from the group comprising calcium stearate, colloidal silicon dioxide, sodium stearyl fumarate, sodium lauryl sulfate, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

In a preferred embodiment of the invention, the composition comprises sodium stearyl fumarate as lubricant. The amount of sodium stearyl fumarate is 0.5-5%, preferably 1-3% by weight of the total composition.

In a preferred embodiment of the invention, the composition comprises colloidal silicon dioxide as glidant. The amount of colloidal silicon dioxide is between 0.5% and 5%, preferably between 1% and 3% by weight of the total composition.

In a preferred embodiment of the invention, the ratio of lubricant to glidant is in the range of 0.1:1 to 5:1, preferably 0.5:1 to 2:1 in order to ensure the desired flowability. Most preferably, this ratio is 1:1.

It has also been found that the ratios of the disintegrant, lubricant and glidant in the composition have also great importance in order to obtain solid oral pharmaceutical compositions providing high solubility and high stability. The ratio of the total amount of disintegrant to the total amount of lubricant and glidant is in the range of from 1.5: 1 to 1:1.

In one embodiment of the invention, the total composition comprises the following:
- 5-60% by weight of sitagliptin maleate
- 5-60% by weight of microcrystalline cellulose
- 10-70% by weight of dibasic calcium phosphate anhydrate
- 1-10% by weight of croscarmellose sodium
- 0.5-5% by weight of sodium stearyl fumarate
- 0.5-5% by weight of colloidal silicon dioxide

In the preferred embodiment of the invention, the compositions disclosed in any of the above embodiments are filled into capsules made of hydroxypropyl methylcellulose (HPMC) material with high moisture resistance.

These analytically selected ratios provide the effective dosages required for the treatment and improve the stability and dissolution profile of the composition subjected to the invention.

According to all these embodiments, the following formulation can be used in the capsule dosage form of the invention.

### Example 1: Capsule formulation

| **Components** | **Amount (%)** |
|---|---|
| Sitagliptin maleate | 15 - 30 |
| Microcrystalline cellulose | 20 - 40 |
| Dibasic calcium phosphate anhydrate | 30 - 45 |
| Croscarmellose sodium | 3 - 7 |
| Sodium stearyl fumarate | 1-3 |
| Colloidal silicon dioxide | 1 - 3 |
| **Total** | **100** |

### Example 2: Capsule formulation

| **Components** | **Amount (%)** |
|---|---|
| Sitagliptin maleate | 22.58 |
| Microcrystalline cellulose | 30.00 |
| Dibasic calcium phosphate anhydrate | 38.42 |
| Croscarmellose sodium | 5.00 |
| Sodium stearyl fumarate | 2.00 |
| Colloidal silicon dioxide | 2.00 |
| **Total** | **100.00** |

The above-mentioned pharmaceutical formulations are prepared by the following steps:
- Sieving sitagliptin maleate, dibasic calcium phosphate anhydrate, microcrystalline cellulose, croscarmellose sodium and colloidal silicon dioxide and then mixing
- Adding sodium stearyl fumarate to this mixture and then mixing
- Filling the obtained mixture into HPMC capsules

## Claims

1. A solid oral pharmaceutical composition comprising at least one DPP-4 inhibitor or a pharmaceutically acceptable salt thereof, wherein the composition is free of polyvinylpyrrolidone and wherein said DPP-4 inhibitor or a pharmaceutically acceptable salt thereof is sitagliptin maleate and wherein the ratio of the total amount of disintegrants to the total amount of lubricant and glidant is in the range of from 1.5:1 to 1:1.

2. The solid oral pharmaceutical composition according to claim 1, wherein the composition is also free of magnesium stearate .

3. The solid oral pharmaceutical composition according to claim 1 or 2, wherein the amount of sitagliptin maleate is in the range of 5-60%, preferably 10-50%, most preferably 15-30% by weight of the total composition.

4. The solid oral pharmaceutical composition according to any one of the preceding claims, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film coated tablet, gastric disintegrating tablet, pill, capsule, oral granule, powder, coated bead system, microsphere, tablet in tablet, inlay tablet, dragee, sachet or orally administrable film.

5. The solid oral pharmaceutical composition according to claim 4, wherein the composition is in the form of capsule.

6. The solid oral pharmaceutical composition according to any one of the preceding claims, wherein the composition comprises at least one excipient selected from fillers, disintegrants, lubricants, glidants or mixtures thereof.

7. The solid oral pharmaceutical composition according to claim 6, wherein the amount of the disintegrant is less than 10%, more preferably less than 7% by weight of the composition.

8. The solid oral pharmaceutical composition according to claim 6, wherein the composition comprises at least one lubricant and at least one glidant selected from the group comprising calcium stearate, colloidal silicon dioxide, sodium stearyl fumarate, sodium lauryl sulfate, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

9. The solid oral pharmaceutical composition according to any one of the claims 6 to 8, wherein the ratio of lubricant to glidant is in the range of 0.1:1 to 5:1, preferably 0.5:1 to 2:1.

10. The solid oral pharmaceutical composition according to any one of the preceding claims, wherein the composition comprises
- 5-60% by weight of sitagliptin maleate
- 5-60% by weight of microcrystalline cellulose
- 10-70% by weight of dibasic calcium phosphate anhydrate
- 1-10% by weight of croscarmellose sodium
- 0.5-5% by weight of sodium stearyl fumarate
- 0.5-5% by weight of colloidal silicon dioxide.

11. A process for preparing the solid oral pharmaceutical composition according to claim 10, wherein the said process comprises the following steps:
- Sieving sitagliptin maleate, dibasic calcium phosphate anhydrate, microcrystalline cellulose, croscarmellose sodium and colloidal silicon dioxide and then mixing
- Adding sodium stearyl fumarate to this mixture and then mixing
- Filling the obtained mixture into HPMC capsules

## Patentansprüche

1. Feste orale pharmazeutische Zusammensetzung, umfassend mindestens einen DPP-4-Inhibitor oder ein pharmazeutisch verträgliches Salz davon, wobei die Zusammensetzung frei von Polyvinylpyrrolidon ist und wobei der DPP-4-Inhibitor oder ein pharmazeutisch verträgliches Salz davon Sitagliptinmaleat ist und wobei das Verhältnis der Gesamtmenge an Desintegrationsmitteln zur Gesamtmenge an Gleitmitteln im Bereich von 1,5:1 bis 1:1 liegt.

2. Feste orale pharmazeutische Zusammensetzung nach Anspruch 1, wobei die Zusammensetzung auch frei von Magnesiumstearat ist.

3. Die feste orale pharmazeutische Zusammensetzung gemäß Anspruch 1 oder 2, wobei die Menge an Sitagliptinmaleat im Bereich von 5 bis 60 Gew.-%, vorzugsweise 10 bis 50 Gew.-%, besonders bevorzugt 15 bis 30 Gew.-% der Gesamtzusammensetzung liegt.

4. Die feste orale pharmazeutische Zusammensetzung gemäß einem der vorhergehenden Ansprüche, wobei die Zusammensetzung in Form einer beschichteten Tablette, einer dreischichtigen Tablette, einer zweischichtigen Tablette, einer mehrschichtigen Tablette, einer oral zerfallenden Tablette, einer Minitablette, Pellets, Zuckerpellets, Bukkaltablette, Sublingualtablette, Brausetablette, Tabletten mit sofortiger Freisetzung, Tabletten mit modifizierter Freisetzung, Filmtablette, magensaftlösliche Tablette, Pille, Kapsel, orales Granulat, Pulver, beschichtetes Perlen-System, Mikrosphäre, Tablette in Tablette, Einlagetablette, Dragee, Sachet oder oral verabreichbarer Film vorliegt.

5. Die feste orale pharmazeutische Zusammensetzung gemäß Anspruch 4, wobei die Zusammensetzung in Form einer Kapsel vorliegt.

6. Die feste orale pharmazeutische Zusammensetzung gemäß einem der vorhergehenden Ansprüche, wobei die Zusammensetzung mindestens einen Hilfsstoff umfasst, der aus Füllstoffen, Sprengmitteln, Gleitmitteln, Fließmitteln oder Mischungen davon ausgewählt ist.

7. Die feste orale pharmazeutische Zusammensetzung nach Anspruch 6, wobei die Menge des Sprengmittels weniger als 10 Gew.-%, vorzugsweise weniger als 7 Gew.-% der Zusammensetzung beträgt.

8. Die feste orale pharmazeutische Zusammensetzung nach Anspruch 6, wobei die Zusammensetzung mindestens ein Gleitmittel und mindestens ein Fließmittel umfasst, ausgewählt aus der Gruppe, bestehend aus Calciumstearat, kolloidalem Siliciumdioxid, Natriumstearylfumarat, Natriumlaurylsulfat, Zinkstearat, Calciumstearat, Mineralöl, Talk, Polyethylenglykol, Glycerylmonostearat, Glycerylpalmitostearat, Magnesiumlaurylsulfat, Fumarsäure, Zinkstearat, Stearinsäure, hydrierte natürliche Öle, Siliciumdioxid, Paraffin oder Mischungen davon ausgewählt ist.

9. Die feste orale pharmazeutische Zusammensetzung gemäß einem der Ansprüche 6 bis 8, wobei das Verhältnis von Gleitmittel zu Fließmittel im Bereich von 0,1:1 bis 5:1, vorzugsweise 0,5:1 bis 2:1 liegt.

10. Die feste orale pharmazeutische Zusammensetzung nach einem der vorhergehenden Ansprüche, wobei die Zusammensetzung umfasst
- 5 bis 60 Gew.-% Sitagliptinmaleat
- 5 bis 60 Gew.-% mikrokristalline Cellulose
- 10 bis 70 Gew.-% dibasisches Calciumphosphat-Anhydrat
- 1 bis 10 Gew.-% Croscarmellose-Natrium
- 0,5 bis 5 Gew.-% Natriumstearylfumarat
- 0,5-5 Gew.-% kolloidales Siliciumdioxid.

11. Verfahren zur Herstellung der festen oralen pharmazeutischen Zusammensetzung gemäß Anspruch 10, wobei das Verfahren die folgenden Schritte umfasst:
- Sieben von Sitagliptinmaleat, dibasischem Calciumphosphat-Anhydrat, mikrokristalliner Cellulose, Croscarmellose-Natrium und kolloidalem Siliciumdioxid und anschließendes Mischen
- Zugabe von Natriumstearylfumarat zu dieser Mischung und anschließendes Mischen
- Füllen der erhaltenen Mischung in HPMC-Kapseln

## Revendications

1. - Composition pharmaceutique orale solide comprenant au moins un inhibiteur de DPP-4 ou un sel pharmaceutiquement acceptable de celui-ci, dans laquelle la composition est exempte de polyvinylpyrrolidone et dans laquelle ledit inhibiteur de DPP-4 ou un sel pharmaceutiquement acceptable de celui-ci est le maléate de sitagliptine et dans laquelle le rapport de la quantité totale de désintégrants à la quantité totale de lubrifiant et d'agent de glissement est dans la plage de 1,5:1 à 1:1.

2. - Composition pharmaceutique orale solide selon la revendication 1, dans laquelle la composition est également exempte de stéarate de magnésium.

3. - Composition pharmaceutique orale solide selon l'une des revendications 1 ou 2, dans laquelle la quantité de maléate de sitagliptine est dans la plage de 5 à 60 %, de préférence de 10 à 50 %, de la façon que l'on préfère le plus de 15 à 30 % en poids de la composition totale.

4. - Composition pharmaceutique orale solide selon l'une quelconque des revendications précédentes, dans laquelle la composition se présente sous forme de comprimés enrobés, comprimés tricouches, comprimés bicouches, comprimés multicouches, comprimés à désintégation orale, mini-comprimés, pastilles, granulés de sucre, comprimés buccaux, comprimés sublinguaux, comprimés effervescents, comprimés à libération immédiate, comprimés à libération modifiée, comprimés pelliculés, comprimés à désintégration gastrique, pilules, gélules, granulés oraux, poudre, système à perles enrobées, microsphères, formes de comprimé-dans-comprimé, comprimés incrustés, dragées, sachets ou films administrable par voie orale.

5. - Composition pharmaceutique orale solide selon la revendication 4, dans laquelle la composition est sous la forme de gélule.

6. - Composition pharmaceutique orale solide selon l'une quelconque des revendications précédentes, dans laquelle la composition comprend au moins un excipient choisi parmi les charges, les désintégrants, les lubrifiants, les agents de glissement ou leurs mélanges.

7. - Composition pharmaceutique orale solide selon la revendication 6, dans laquelle la quantité du désintégrant est inférieure à 10%, de façon davantage préférée inférieure à 7% en poids de la composition.

8. - Composition pharmaceutique orale solide selon la revendication 6, dans laquelle la composition comprend au moins un lubrifiant et au moins un agent de glissement choisis dans le groupe comprenant le stéarate de calcium, le dioxyde de silicium colloïdal, le fumarate de stéaryle sodique, le lauryl sulfate de sodium, le stéarate de zinc, le stéarate de calcium, l'huile minérale, le talc, le polyéthylène glycol, le monostéarate de glycéryle, le palmitostéarate de glycéryle, le lauryl sulfate de magnésium, l'acide fumarique, le stéarate de zinc, l'acide stéarique, les huiles naturelles hydrogénées, la silice, la paraffine ou leurs mélanges.

9. - Composition pharmaceutique orale solide selon l'une quelconque des revendications 6 à 8, dans laquelle le rapport du lubrifiant à l'agent de glissement est dans la plage de 0,1:1 à 5:1, de préférence de 0,5:1 à 2:1.

10. - Composition pharmaceutique orale solide selon l'une quelconque des revendications précédentes, dans laquelle la composition comprend
- 5-60% en poids de maléate de sitagliptine
- 5-60% en poids de cellulose microcristalline
- 10-70% en poids de phosphate de calcium dibasique anhydrate
- 1-10% en poids de croscarmellose sodique
- 0,5-5% en poids de fumarate de sodium et de stéaryle
- 0,5-5% en poids de dioxyde de silicium colloïdal.

11. - Procédé de préparation de la composition pharmaceutique orale solide selon la revendication 10, dans lequel ledit procédé comprend les étapes suivantes :
- tamiser le maléate de sitagliptine, le phosphate de calcium dibasique anhydrate, la cellulose microcristalline, la croscarmellose sodique et le dioxyde de silicium colloïdal, puis mélanger ;
- ajouter le fumarate de sodium et de stéaryle à ce mélange, puis mélanger.
- remplir du mélange obtenu des gélules de HPMC
